# EUROPEAN PATENT SPECIFICATION

(11) **EP 3 193 832 B1**
(45) Date of publication and mention of the grant of the patent: **07.08.2019**
(21) Application number: 16745816.5
(22) Date of filing: 15.06.2016
(51) Int. Cl.: A61K 9/00, A61K 9/08, A61K 47/36, A61K 47/38

(54) **VISCOELASTIC PREPARATION FOR USE IN SURGICAL METHODS OF OPHTHALMIC SURGERY**
VISKOELASTISCHE ZUBEREITUNG ZUR VERWENDUNG IN CHIRURGISCHEN VERFAHREN DER AUGENCHIRURGIE
PRÉPARATION VISCOÉLASTIQUE DESTINÉE À UNE UTILISATION DANS DES TECHNIQUES CHIRURGICALES EN CHIRURGIE OPHTALMOLOGIQUE

(30) Priority: 17.06.2015 IT UB20151442
(43) Date of publication of application: 26.07.2017
(73) Proprietor: AL.CHI.MI.A. S.r.l., 35020 Ponte S. Nicolò (Padova) (IT)
(72) Inventor: BECCARO, Mauro, 35010 Cadoneghe (PD) (IT); BETTINI, Enrico, 30032 Fiesso d'Artico (VE) (IT); SIGNORI, Paolo, 37100 Verona (IT)
(74) Representative: Ponchiroli, Simone
(86) International application number: PCT/IB2016/053518
(87) International publication number: WO 2016/203381

(56) References cited:
- WO-A2-2012/117115
- CA-A1- 2 726 086
- JP-A- H 111 418

## Description

### Technical field

This invention relates to a viscoelastic preparation for use in methods for the treatment of the bodies of humans or animals and a ready-to-use single-dose container that contains it.

This invention was devised in particular with reference to the surgical methods of ophthalmic surgery which necessitate constant protection and hydration of the cornea during the surgery to prevent it from being accidentally damaged.

### Background art

According to the traditional surgical technique, protection and hydration of the cornea was guaranteed exclusively by regularly and repeatedly irrigating it with a physiological saline solution during the surgery.

Then, the article by R. M. Kershner "6 Tips to Clear Cornea", Cataract Surgery, April 1999, to guarantee cornea hydration suggested the use of a viscoelastic preparation comprising hydroxypropyl methylcellulose (hereinafter referred to as HPMC), which also guaranteed the benefit of an optical enlargement of the visual field of the surgeon. Application of the viscoelastic preparation on the cornea in fact caused the formation on the cornea itself of a protective layer that as well as guaranteeing hydration, also acted as a magnifying glass.

The same idea was subsequently taken up in the commercial product HPMC IN BALANCED SALT SOLUTION made and marketed by Moorfields Pharmaceuticals of London, United Kingdom, and in patent application WO 2012/117115, as well as in the commercial product corresponding to the latter patent application, CORNEA PROTECT® marketed by Croma-Pharma Gmbh of Leobendorf, Austria.

Considering the use that must be made of them, such viscoelastic preparations should comply with some specifications both regarding pH values and osmolarity (so as to be physiologically safe) and in terms of their transmittance and viscosity physical parameters.

However, this prior art technology has several disadvantages.

In particular, the Applicant was able to verify that different samples, even from the same batch, of a viscoelastic preparation made in accordance with the prior art, could have significantly different properties and that, in particular, in some cases the characteristics of the preparation at the time of use showed a general decline such that it rendered that specific sample unsuitable for the planned use. The decline affected in particular the pH, transmittance, osmolality and dynamic viscosity values.

Although the appearance of these problems would seem, at first sight, inexplicable and random, subsequent precision tests allowed the Applicant to understand that the decline in performance occurred systematically depending on the methods of preservation that had been used for the samples.

In fact, it should be considered that methods for the preservation of preparations of this type may be divided into two major categories in terms of temperature: on one hand preservation at a controlled temperature, and on the other hand preservation at ambient temperature.

Regarding preservation at a controlled temperature, this may normally be either at a temperature of approximately 4°C or at a temperature of approximately -20°C.

In contrast, preservation at ambient temperature may, in use, expose products to significant temperate changes during their transportation from the production site to the place where they will be used. In fact, transportation is usually in the compartments of vehicles without any kind of air conditioning or climate control. This means that, depending on the location, in winter the products may be exposed to low or very low temperatures (even well below zero degrees) and in summer to high or very high temperatures (easily as high as 50°C).

What it was possible to ascertain was that there were the greatest declines in performance both with preservation at a controlled temperature of -20°C, and with preservation at ambient temperature when the products were accidentally exposed to temperatures which were either excessively low (comparable to -20°C) or excessively high (approximately 50°C).

Second, the viscoelastic preparations made in accordance with the prior art are able to remain on the cornea for a relatively limited time. This means that, in the case of longer surgeries, subsequent applications of the viscoelastic preparation on the cornea may be required.

A further disadvantage relates to the ready-to-use single-dose containers, in which the type of viscoelastic preparation to which this invention relates are currently marketed. In fact, many surgeons have expressed to the Applicant a certain disappointment regarding the practicality of the squeezable single-dose containers currently on the market, which would not allow the viscoelastic preparation to be applied in the best possible way (both in terms of application precision and in terms of quantity).

### Disclosure of the invention

In this context, the technical purpose which forms the basis of this invention is to provide a viscoelastic preparation for use in methods for the treatment of the bodies of humans or animals, in particular in surgical methods of ophthalmic surgery, and a ready-to-use single-dose container that contains it, which overcome the above-mentioned disadvantages.

In particular, the technical purpose of this invention is to provide a viscoelastic preparation for use in methods for the treatment of the bodies of humans or animals, in particular in surgical methods of ophthalmic surgery, which is more stable than the prior art preparations following all methods of preservation.

A further technical purpose of this invention is to provide a viscoelastic preparation for use in methods for the treatment of the bodies of humans or animals, in particular in surgical methods of ophthalmic surgery, which can remain on the cornea for longer than the prior art preparations.

Another technical purpose of this invention is to provide a viscoelastic preparation for use in methods for the treatment of the bodies of humans or animals, in particular in surgical methods of ophthalmic surgery, which can be easily and quickly removed after surgery.

Last but not least, the technical purpose of this invention is to provide a ready-to-use single-dose container that contains the viscoelastic preparation of this invention and that allows improved application of it (both in terms of positioning precision and in terms of quantity), better than the prior art containers.

The technical purpose and the aims specified are substantially achieved by a viscoelastic preparation for use in methods for the treatment of the bodies of humans or animals, in particular in surgical methods of ophthalmic surgery, and by a ready-to-use single-dose container that contains it, as described in the appended claims.

### Brief description of the drawings

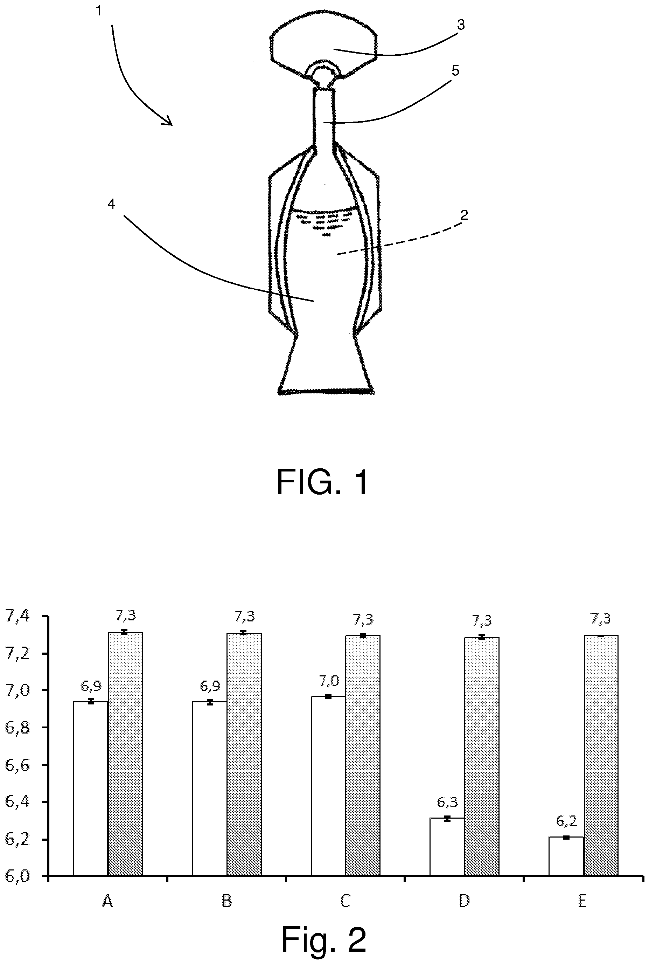

Further features and advantages of this invention are more apparent in the detailed description of a preferred, non-limiting embodiment of a viscoelastic preparation for use in methods for the treatment of the bodies of humans or animals, in particular in surgical methods of ophthalmic surgery, and of a ready-to-use single-dose container that contains it illustrated in the appended Figure 1.

### Detailed description of preferred embodiments of the invention

The invention relates to a viscoelastic preparation for use according to claim 1 and to a ready-to-use single-dose container according to claim 13.

It should be noticed that the context of this invention includes the viscoelastic preparation for use in a surgical method of ophthalmic surgery.

Regarding the viscoelastic preparation, similarly to that described in WO 2012/117115, even the preparation according to this invention is, as already indicated, intended for use in methods for the treatment of the bodies of humans or animals, in particular in a surgical method of ophthalmic surgery. Moreover, similarly to the prior art preparations, even the preparation according to this invention comprises a water-based phosphate buffer and at least one polymer for increasing the viscosity selected from the group comprising hydroxypropyl methylcellulose HPMC, carboxymethylcellulose, hydroxyethylcellulose, hyaluronic acid, sodium alginate, hydroxypropyl-guar polyvinylpyrrolidone, polyvinyl alcohol, polymethacrylic acid, copolymer with polyoxyethylene-polyoxypropylene and polyethylene glycol.

However, according to this invention the polymer for increasing viscosity is present in a quantity of between 0.2% and 20%. Moreover, also according to this invention, in addition to the above-mentioned polymer for increasing the viscosity, the viscoelastic preparation comprises at least two other main components, that is to say, xanthan gum in a quantity of between 0.01% and 1%, and carrageenan in a quantity of between 0.005% and 0.5%.

Moreover, in a preferred embodiment of this invention, the viscoelastic preparation comprises, as a weight/volume percentage, the at least one polymer for increasing the viscosity in a quantity of between 1% and 5%, xanthan gum in a quantity of between 0.05% and 1%, and carrageenan in a quantity of between 0.025% and 0.5%.

Furthermore, amongst the various possible polymers for increasing the viscosity, the one that is greatly preferred is hydroxypropyl methylcellulose HPMC, which in the preferred embodiments is therefore used either as the only polymer or as the main polymer.

As regards the phosphate buffer, many different formulations are possible, depending on requirements. On each occasion the expert may therefore use the one best suited to the purpose. However, in the preferred embodiments, the phosphate buffer is water-based and comprises at least sodium chloride, anhydrous dibasic sodium phosphate and monohydrate monobasic sodium phosphate.

In particular, as a weight/volume percentage, it advantageously comprises at least:
- sodium chloride in a quantity of between 0.4% and 1.6%;
- anhydrous dibasic sodium phosphate in a quantity of between 0.07% and 0.3%; and
- monohydrate monobasic sodium phosphate in a quantity of between 0.01% and 0.05%.

In the preferred embodiments, the quantities of each of the three main components and of the various components of the phosphate buffer are selected in such a way that, at a temperature of 25°C the viscoelastic preparation has a pH of between 7.1 and 7.5, and/or an osmolality of between 260 and 319 mOsm/kg, and/or a transmittance at 600 nm that is greater than or equal to 90% of that of deionised water H₂O, and/or an oscillatory dynamic viscosity (measured under the conditions indicated below) of between 3200 and 5000 mPa·s. As regards requirements relating to osmolality, it should be noticed that, in the known way, it is possible to adjust this value by simply varying the quantity of sodium chloride used in the phosphate buffer, without this being able to have any significant effects on the other properties of interest of the preparation.

Particularly when the intended use in is methods of ophthalmic surgery, at least in a range of temperatures between 5°C and 40°C, the viscoelastic preparation is in the form of a gel with pseudoplastic behaviour.

Finally, it should be noticed that, if necessary, the viscoelastic preparation disclosed may also comprise further components, even medicines.

Concerning the preferred uses of the viscoelastic preparation according to this invention, as already indicated, the one for which it was provided is use for hydrating and protecting the cornea during a surgical method of ophthalmic surgery. For that purpose, a predetermined quantity of the viscoelastic preparation (usually not more than 2 ml) is applied on the surface of the cornea and in a very short time distributes itself substantially over the entire exposed surface of the cornea, forming a thin protective layer.

Once it has covered the cornea, similarly to the corresponding prior art preparations, the viscoelastic preparation disclosed also guarantees the surgeon an optical enlargement effect of the visual field observable through the cornea (in this way, it is easier for the surgeon to see the internal structures of the eye).

At the end of the surgery, the preparation can be removed easily and quickly by simply washing the surface of the cornea with at least 20 ml of BSS in a continuous stream (advantageously using a syringe with a corneal irrigation cannula).

Below are some experimental results of comparative tests performed on samples of viscoelastic preparations CORNEA PROTECT® marketed by Croma-Pharma Gmbh of Leobendorf, Austria, and on samples from three different production batches of a viscoelastic preparation according to this invention, having the following formulation:

| **PREPARATION Component name:** | **% quantity (w/v)** | **Theoretical quantity** |
|---|---|---|
| Hydroxypropyl methylcellulose (HPMC 60HD 4000 cps marketed by Giusto Faravelli Spa of Milan, Italy...) | 2.00% | 4.00 g |
| Xanthan gum (XANTURAL® 75 marketed by CP Kelco of Atlanta - USA) | 0.10% | 0.20 g |
| Carrageenan (GENUVISCO® CARRAGEENAN CG-131 marketed by CP Kelco of Atlanta - USA) | 0.05% | 0.10 g |
| Phosphate buffer | - | q.s. to 200 ml |

| **Material for preparation of the phosphate buffer** | **% quantity (w/v)** | **Theoretical quantity** |
|---|---|---|
| NaCl | 0.820 | 1.64065 g |
| Na2HPO4 | 0.151 | 0.3026 g |
| NaH2PO4 | 0.02657 | 0.05314 g |
| H2O USP/WI | q.s. 200 ml | q.s. 200 ml |

The aim of the tests was to measure the pH, transmittance and oscillatory dynamic viscosity at 25°C, before and after preservation of each sample at a given temperature for a predetermined time.

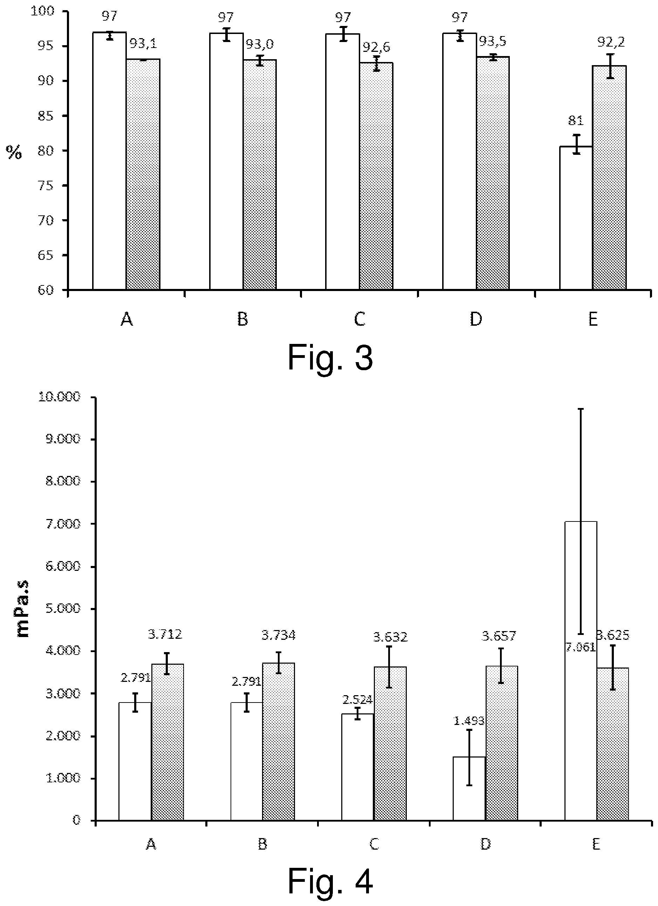

The results obtained are highlighted in the accompanying figures 2 to 4, which relate respectively to the pH, the transmittance and the oscillatory dynamic viscosity with reference to the following cases (also labelled in the figures with the respective upper case letter from A to E):
- A: measurement taken before starting the preservation period;
- B: measurement taken after 14 day of preservation at +20°C ± 5°C;
- C: measurement taken after 15 days of preservation at +40°C ± 2°C;
- D: measurement taken after 3 days of preservation at +50°C ± 2°C; and
- E: measurement taken after 3 days of preservation at -20°C ± 5°C.

The oscillatory dynamic viscosity measurements were taken using a Anton Paar Physica 301 rheometer and for all of the analyses a plate - cone configuration was used, with a CP60-1/TI/S titanium measuring head (cone diameter 60 mm, 1° top angle, 0.025 mm gap). The measurement was taken in "stress sweep" mode with the following parameters: temperature = 20-25°C (controlled using a Peltier cell, code P-PTD-200+ H-PTD-200); constant oscillation frequency = 1 Hz; torque range: 0.1 - 1000 µNm (with logarithmic variation); number of measuring points = 20 (with a measuring time of 5 - 10 minutes).

The dynamic viscosity was then calculated as the average of the values of the actual viscosity expressed in [mPa·s] supplied by the instrument for the twenty measuring points.

Regarding the three batches of preparation made according to this invention, the production method used involves solubilisation of the components using a gradual process requiring the precise sequence of steps described in the table below, which indicates both the time/temperature conditions and those relating to the stirring speed.

| **Step of solubilisation of components** | **Temperature (°C)** | | | **Time** | **Stirring Speed (rpm)** |
|---|---|---|---|---|---|
| | **Average** | **Min.** | **Max.** | | |
| Step 0 Bring the phosphate buffer to approximately 80°C | 81.9 | 80.0 | 90.0 | 5-10 min. | no |
| Step 1 solubilisation of HPMC | 81.5 | 80.0 | 90.0 | 30 min. | 800 |
| Step 2 solubilisation of Xanthan Gum | 66.8 | 65.0 | 80.0 | 45 min. | 800 |
| Step 3 solubilisation of carrageenan | 60.6 | 55.0 | 65.0 | 30 min. | 800 |
| Step 4 continue until complete solubilisation of the components and ambient temperature has been reached | 29.6 | 25.0 | 55.0 | 2- 4h | 800 |

In the accompanying figures, for each case the left-hand bar (white) relates to the comparison preparation, whilst the right-hand bar (shaded grey) relates to the average of the measurements taken on the three samples of the preparation according to this invention, taken from three separate production batches. For each set of data the bars indicating, respectively, standard measuring errors regarding the preparation according to this invention and standard deviation regarding the comparison preparation were highlighted.

As can be seen in the graphs shown in Figures 2 to 4, the behaviour of the preparations made in accordance with this invention proved to be better than that of the commercial comparison preparation.

In particular, whilst all of the viscoelastic preparations according to this invention had substantially constant pH values irrespective of the preservation conditions, the comparison preparation showed an obvious decline in the pH value following preservation in conditions D and E. Furthermore, the comparison preparation showed a pH that, even at the start, was significantly lower and not entirely satisfactory for use in methods of ophthalmic surgery, since it was also below the tear film physiological pH fluctuation range (which in literature is considered to be between 7.14 and 7.82). In contrast, all of the batches made according to this invention showed a pH value that was always within the range corresponding to the physiological values of the tear film.

Moving on to transmittance, although the values of the preparations made in accordance with this invention were slightly lower than the maximum values achieved by the commercial comparison preparation, they were still more than good enough for use of the preparation as a cornea protector, but above all, they remained constant following all methods of preservation, unlike the comparison preparation which showed a noticeable decline in case E.

Finally, regarding oscillatory dynamic viscosity, the preparations made in accordance with this invention not only showed stable behaviour following all methods of preservation, but also had significantly higher values than the commercial comparison preparation, which moreover, showed a significant decline in viscosity in case D and a significant increase in it in case E. In both of the latter cases D, E, of preservation in extreme conditions, the variation in viscosity measured for the comparison preparation was excessive and indicated that there had been a structural alteration in the preparation.

Moving on to the single-dose container according to this invention, in Figure 1 it is labelled 1 whilst the viscoelastic preparation contained in it is labelled 2.

Advantageously, the single-dose container is ready to use and contains at least 2 ml of sterile viscoelastic preparation.

In accordance with a first embodiment illustrated in Figure 1, the container 1 is of the squeezable type and is equipped with a closing cap 3 (which may be definitively separated by breaking as in Figure 1, or which may be taken off and put back on in other embodiments). Once the cap 3 has been removed, the main body 4 of the container 1 can simply be squeezed with the fingers to cause the viscoelastic preparation to come out. That result may be obtained by using a material such as PURELL RP270G (polypropylene, random copolymer) to make the container 1.

Advantageously, the container 1 has an internal volume that is greater than the volume of the viscoelastic preparation contained in it, to facilitate management of it once opened (volumes of around 3 ml for 2 ml of viscoelastic preparation are considered optimum).

In an alternative embodiment, the single-dose container may also be constituted of a filled and capped syringe, with or without an additional tube or cannula that can be mounted on the nozzle of the syringe body after removing the cap.

However, advantageously, at one end of it the single-dose container 1 comprises an elongate supplying portion 5 to which the closing cap 3 is applied, and which has, transversally to the line along which it is elongate, a smaller dimension. In the case illustrated in Figure 1 it is constituted of a tubular portion of the container 1, whilst in the case of the syringe it may be constituted either of the syringe nozzle or the cannula, if present. In this way, in use, the free end of the elongate portion can easily be brought near to the cornea for application of the viscoelastic preparation. Preferably the elongate portion extends for at least 1 cm.

This invention brings important advantages.

In fact, first, it was possible to provide a viscoelastic preparation for use in methods for the treatment of the bodies of humans or animals which is much more stable than prior art preparations relative to variations in the preservation temperature.

Secondo, initial practical tests carried out seem to indicate that the viscoelastic preparation according to this invention can guarantee a time for which it remains on the cornea that is longer than those of prior art preparations, so that it should always be possible to perform the whole surgery without the need for subsequent applications of the preparation.

However, at the same time, the preparation made according to this invention is easily and quickly removable after the surgery.

In turn, even the ready-to-use single-dose container according to this invention, when equipped with the elongate portion, allows improved application of the preparation on the cornea compared with prior art containers.

Finally, it should be noticed that this invention is relatively easy to produce and that even the cost linked to implementing the invention is not very high.

## Claims

1. A viscoelastic preparation for use in a surgical method of ophthalmic surgery , comprising a water-based phosphate buffer and, as a weight/volume percentage, at least:
- at least one polymer for increasing the viscosity selected from the group consisting of hydroxypropyl methylcellulose HPMC, carboxymethylcellulose, hydroxyethylcellulose, hyaluronic acid, sodium alginate, hydroxypropyl-guar polyvinylpyrrolidone, polyvinyl alcohol, polymethacrylic acid, copolymer with polyoxyethylene-polyoxypropylene and polyethylene glycol, in a quantity of between 0.2% and 20%;
- xanthan gum in a quantity of between 0.01% and 1%;
- carrageenan in a quantity of between 0.005% and 0.5%.

2. The viscoelastic preparation for use in a surgical method of ophthalmic surgery according to claim 1, wherein the viscoelastic preparation acts as a hydrating and protective substance for the cornea during the surgical method.

3. The viscoelastic preparation for use in a surgical method of ophthalmic surgery according to claim 2, wherein the preparation forms a protective layer on the cornea and guarantees an optical enlargement effect of the visual field observable through the cornea.

4. The viscoelastic preparation for use according to any one of claims 1 to 3, comprising as a weight/volume percentage, at least:
- said at least one polymer for increasing the viscosity in a quantity of between 1% and 5%;
- xanthan gum in a quantity of between 0.05% and 1%;
- carrageenan in a quantity of between 0.025% and 0.5%.

5. The viscoelastic preparation for use according to any one of claims 1 to 4,
wherein the polymer for increasing the viscosity is only or mainly hydroxypropyl methylcellulose HPMC.

6. The viscoelastic preparation for use according to any one of claims 1 to 5,
wherein the water-based phosphate buffer comprises at least sodium chloride, anhydrous dibasic sodium phosphate and monohydrate monobasic sodium phosphate.

7. The viscoelastic preparation for use according to claim 6, wherein the water-based phosphate buffer comprises as a weight/volume percentage at least:
- sodium chloride in a quantity of between 0.4% and 1.6%;
- anhydrous dibasic sodium phosphate in a quantity of between 0.07% and 0.3%; and
- monohydrate monobasic sodium phosphate in a quantity of between 0.01% and 0.05%.

8. The viscoelastic preparation for use according to any one of claims 1 to 7, **characterised in that**, at a temperature of 25°C, it has a pH of between 7.1 and 7.5.

9. The viscoelastic preparation for use according to any one of claims 1 to 8, **characterised in that**, at a temperature of 25°C, it has an osmolality of between 260 and 319 mOsm/kg.

10. The viscoelastic preparation for use according to any one of claims 1 to 9, **characterised in that**, at a temperature of 25°C, it has a transmittance at 600 nm that is greater than or equal to 90% of that of deionised water.

11. The viscoelastic preparation for use according to any one of claims 1 to 10, **characterised in that**, at a temperature of 25°C, it has an oscillatory dynamic viscosity of between 3200 and 5000 mPa·s.

12. The viscoelastic preparation for use according to any one of claims 1 to 11, **characterised in that** it is liquid at least in a temperature range of between 5°C and 40°C.

13. A ready-to-use single-dose container containing at least 2 ml of a sterile viscoelastic preparation according to any one of claims 1 to 12.

14. The single-dose container according to claim 13, **characterised in that** it is squeezable or **in that** it is constituted of a capped syringe.

15. The single-dose container according to claim 13 or 14, **characterised in that** it comprises at one end of it an elongate supplying portion (5), which is closed at a free end by a removable closing cap (3).

## Patentansprüche

1. Eine viskoelastische Zubereitung zur Verwendung in einem chirurgischen Verfahren der Augenchirurgie, einen Phosphatpuffer auf Wasserbasis und, als Gewichts-/Volumensprozentsatz, zumindest Folgendes enthaltend:
- Mindestens ein Polymer zur Erhöhung der Viskosität, ausgewählt aus der Gruppe bestehend aus Hydroxypropylmethylcellulose (HPMC), Carboxymethylcellulose, Hydroxyethylcellulose, Hyaluronsäure, Natriumalginat, Hydroxypropyl-Guar Polyvinylpyrrolidon, Polyvinylalkohol, Polymethacrylsäure, Copolymer mit Polyoxyethylen-Polyoxypropylen und Polyethylenglykol in einer Menge zwischen 0,2 % und 20 %;
- Xanthangummi in einer Menge zwischen 0,01 % und 1 %;
- Carrageen in einer Menge zwischen 0,005 % und 0,5 %.

2. Die viskoelastische Zubereitung zur Verwendung in einem chirurgischen Verfahren der Augenchirurgie nach Patentanspruch 1, wobei die viskoelastische Zubereitung als hydratisierender und schützender Stoff für die Hornhaut während des chirurgischen Verfahrens dient.

3. Die viskoelastische Zubereitung zur Verwendung in einem chirurgischen Verfahren der Augenchirurgie nach Patentanspruch 2, wobei die Zubereitung eine Schutzschicht auf der Hornhaut bildet und einen optischen Vergrößerungseffekt des durch die Hornhaut wahrnehmbaren Sichtfelds garantiert.

4. Die viskoelastische Zubereitung zur Verwendung nach jedem der Patentansprüche 1 bis 3, als Gewichts-/Volumensprozentsatz zumindest Folgendes enthaltend:
- Besagtes mindestens ein Polymer zur Erhöhung der Viskosität in einer Menge zwischen 1 % und 5 %;
- Xanthangummi in einer Menge zwischen 0,05 % und 1 %;
- Carrageen in einer Menge zwischen 0,025 % und 0,5 %.

5. Die viskoelastische Zubereitung zur Verwendung nach jedem der Patentansprüche 1 bis 4, wobei das Polymer zur Erhöhung der Viskosität nur oder hauptsächlich Hydroxypropylmethylcellulose (HPMC) ist.

6. Die viskoelastische Zubereitung zur Verwendung nach jedem der Patentansprüche 1 bis 5, wobei der Phosphatpuffer auf Wasserbasis mindestens Natriumchlorid, dibasisches Natriumphosphat wasserfrei und monobasisches Natriumphosphat-Monohydrat enthält.

7. Die viskoelastische Zubereitung zur Verwendung nach dem Patentanspruch 6, wobei der Phosphatpuffer auf Wasserbasis als Gewichts-/Volumensprozensatz mindestens Folgendes enthält:
- Natriumchlorid in einer Menge zwischen 0,4 % und 1,6 %;
- Dibasisches Natriumphosphat wasserfrei in einer Menge zwischen 0,07 % und 0,3 %; und
- Monobasisches Natriumphosphat-Monohydrat in einer Menge zwischen 0,01 % und 0,05 %.

8. Die viskoelastische Zubereitung zur Verwendung nach jedem der Patentansprüche 1 bis 7, **gekennzeichnet dadurch, dass** sie bei einer Temperatur von 25° C einen pH-Wert zwischen 7,1 und 7,5 hat.

9. Die viskoelastische Zubereitung zur Verwendung nach jedem der Patentansprüche 1 bis 8, **gekennzeichnet dadurch, dass** sie bei einer Temperatur von 25° C eine Osmolalität zwischen 260 und 319 mosm/kg hat.

10. Die viskoelastische Zubereitung zur Verwendung nach jedem der Patentansprüche 1 bis 9, **gekennzeichnet dadurch, dass** sie bei einer Temperatur von 25° C eine Durchlässigkeit (Transmission) von 600 nm hat, die größer oder gleich 90 % derjenigen von deionisiertem Wasser ist.

11. Die viskoelastische Zubereitung zur Verwendung nach jedem der Patentansprüche 1 bis 10, **gekennzeichnet dadurch, dass** sie bei einer Temperatur von 25° C eine oszillatorisch-dynamische Viskosität zwischen 3200 und 5000 mPa s hat.

12. Die viskoelastische Zubereitung zur Verwendung nach jedem der Patentansprüche 1 bis 11, **gekennzeichnet dadurch, dass** sie zumindest in einem Temperaturbereich zwischen 5° C und 40° C flüssig ist.

13. Ein gebrauchsfertiger Einzeldosis-Behälter, der mindestens 2 ml einer sterilen viskoelastischen Zubereitung nach jedem der Patentansprüche 1 bis 12 enthält.

14. Der Einzeldosis-Behälter nach Patentanspruch 13, **gekennzeichnet dadurch, dass** er ausgedrückt werden kann oder dass er aus einer Spritze mit Kappe besteht.

15. Der Einzeldosis-Behälter nach dem Patentanspruch 13 oder 14, **gekennzeichnet dadurch, dass** er an einem Ende einen länglichen Ausgabeabschnitt (5) umfasst, der an einem freien Ende durch eine abnehmbare Verschlusskappe (3) verschlossen ist.

## Revendications

1. Une préparation viscoélastique destinée à être utilisée dans un procédé chirurgical de chirurgie ophtalmologique, comprenant un tampon phosphate à base d'eau et, en pourcentage poids/volume, au moins :
- au moins un polymère pour augmenter la viscosité choisi dans le groupe constitué par l'hydroxypropylméthylcellulose HPMC, la carboxyméthylcellulose, l'hydroxyéthylcellulose, l'acide hyaluronique, l'alginate de sodium, l'hydroxypropyl-guar et la polyvinylpyrrolidone, l'alcool polyvinylique, l'acide polyméthacrylique, le copolymère avec polyoxyéthylène-polyoxypropylène et polyéthylèneglycol, dans une quantité comprise entre 0,2 % et 20 % ;
- gomme de xanthane dans une quantité comprise entre 0,01 % et 1 % ;
- carraghénine dans une quantité comprise entre 0,005 % et 0,5 %.

2. La préparation viscoélastique destinée à être utilisée dans un procédé chirurgical de chirurgie ophtalmologique selon la revendication 1, dans laquelle la préparation viscoélastique agit en tant que substance hydratante et protectrice de la cornée pendant le procédé chirurgical.

3. La préparation viscoélastique destinée à être utilisée dans un procédé chirurgical de chirurgie ophtalmologique selon la revendication 2, dans laquelle la préparation forme une couche protectrice sur la cornée et garantit un effet d'agrandissement optique du champ visuel pouvant être observé à travers la cornée.

4. La préparation viscoélastique destinée à être utilisée selon l'une quelconque des revendications de 1 à 3, comprenant en pourcentage poids/volume, au moins :
- ledit au moins un polymère pour augmenter la viscosité dans une quantité comprise entre 1 % et 5 % ;
- gomme de xanthane dans une quantité comprise entre 0,05 % et 1 % ;
- carraghénine dans une quantité comprise entre 0,025 % et 0,5 %.

5. La préparation viscoélastique destinée à être utilisée selon l'une quelconque des revendications de 1 à 4, dans laquelle le polymère pour augmenter la viscosité est uniquement ou principalement de l'hydroxypropylméthylcellulose HPMC.

6. La préparation viscoélastique destinée à être utilisée selon l'une quelconque des revendications de 1 à 5, dans laquelle le tampon phosphate à base d'eau comprend au moins du chlorure de sodium, du phosphate de sodium dibasique anhydre et du phosphate de sodium monobasique monohydraté.

7. La préparation viscoélastique destinée à être utilisée selon la revendication 6, dans laquelle le tampon phosphate à base d'eau comprend en pourcentage poids/volume au moins :
- chlorure de sodium dans une quantité comprise entre 0,4 % et 1,6 % ;
- phosphate de sodium dibasique anhydre dans une quantité comprise entre 0,07 % et 0,3 % ; et
- phosphate de sodium monobasique monohydraté dans une quantité comprise entre 0,01 % et 0,05 %.

8. La préparation viscoélastique destinée à être utilisée selon l'une quelconque des revendications de 1 à 7, **caractérisée en ce que**, à une température de 25°C, elle a un pH compris entre 7,1 et 7,5.

9. La préparation viscoélastique destinée à être utilisée selon l'une quelconque des revendications de 1 à 8, **caractérisée en ce que**, à une température de 25°C, elle a une osmolalité comprise entre 260 et 319 mOsm/kg.

10. La préparation viscoélastique destinée à être utilisée selon l'une quelconque des revendications de 1 à 9, **caractérisée en ce que**, à une température de 25°C, elle a un facteur de transmission à 600 nm qui est supérieur ou égal à 90 % de celui de l'eau désionisée.

11. La préparation viscoélastique destinée à être utilisée selon l'une quelconque des revendications de 1 à 10, **caractérisée en ce que**, à une température de 25°C, elle a une viscosité dynamique oscillante comprise entre 3200 et 5000 mPa·s.

12. La préparation viscoélastique destinée à être utilisée selon l'une quelconque des revendications de 1 à 11, **caractérisée en ce qu'**elle est liquide au moins dans une plage de température allant de 5°C à 40°C.

13. Un contenant monodose prêt à l'emploi contenant au moins 2 ml d'une préparation viscoélastique stérile selon l'une quelconque des revendications de 1 à 12.

14. Le contenant monodose selon la revendication 13, **caractérisé en ce qu'**il est compressible ou **en ce qu'**il est constitué d'une seringue capsulée.

15. Le contenant monodose selon la revendication 13 ou 14, **caractérisé en ce qu'**il comprend à une extrémité respective une portion allongée d'alimentation (5), qui est fermée à une extrémité libre par un capuchon de fermeture (3) amovible.
